# EUROPEAN PATENT SPECIFICATION

(11) **EP 2 394 689 B1**
(45) Date of publication and mention of the grant of the patent: **28.06.2017**
(21) Application number: 11164574.3
(22) Date of filing: 03.05.2011
(51) Int. Cl.: A61M 25/01, A61M 25/10, A61M 25/00

(54) **Storage device for balloon catheter**
Lagervorrichtung für einen Ballonkatheter
Dispositif de stockage pour cathéter à ballonnet

(30) Priority: 11.06.2010 JP 2010133944
(43) Date of publication of application: 14.12.2011
(73) Proprietor: ASAHI INTECC CO., LTD., Nagoya-shi, Aichi 463-0024 (JP)
(72) Inventor: Ikegaya, Michihiro, Nagoya-shi Aichi 463-0024 (JP); Iida, Azusa, Nagoya-shi Aichi 451-0053 (JP)
(74) Representative: Winter, Brandl, Fürniss, Hübner, Röss, Kaiser, Polte - Partnerschaft mbB

(56) References cited:
- EP-A1- 0 228 787
- US-A- 5 242 399
- US-A- 5 352 236
- US-A- 6 110 146
- US-B1- 7 850 643

## Description

### Technical Field

The present invention relates to a storage device for a balloon catheter, having a fixture for a stylet.

### Background Art

Conventionally, a balloon catheter is used to dilate a stenosis or the like inside a body cavity such as a vessel. Examples of the balloon catheter include a so-called rapid exchange type having a relatively short guidewire lumen for enabling quick replacement of a guidewire inserted through the balloon catheter (see, for example, JP-A-10-503386). The rapid exchange type balloon catheter has an opening of an inner shaft constituting the guidewire lumen on a side surface of an outer shaft arranged at the rear side of a balloon. The inner shaft constituting the guidewire lumen is arranged only at a front side portion of the catheter.

A front side portion of such a rapid exchange type balloon catheter, where the guidewire lumen is present, is more flexible than a rear side portion thereof. This is because a wire called core wire for adjusting stiffness of the catheter is inserted into the rear side of the outer shaft not provided with the guidewire lumen, or because a hard resin is used as the material for the rear side portion of the outer shaft.

To prevent bending of the front side portion where the guidewire lumen is present, or to prevent kinking of the guidewire lumen, a core material called stylet is inserted into the guidewire lumen over substantially the entire length thereof in the balloon catheter before use.

Also, the balloon yet to be dilated is appropriately folded in the balloon catheter before use. To maintain this folded state, the balloon is inserted into a cylindrical member called a balloon cover (see, for example, U.S. Patent No. 5,342,307).

Furthermore, such a balloon catheter before use is accommodated in a circularly-wound tubular accommodating device. A connector attached to a rear end of the balloon catheter is held in a connector holder fixed to the accommodating device (see, for example, JP-A-2004-290395).

Further, US 5,352,236 discloses a storage device for a balloon catheter, in accordance with the preamble claim 1.

When an operator such as a physician uses the balloon catheter described above, the connector is detached from the connector holder and the catheter is pulled out of the tubular accommodating device. Upon the pullout, the stylet inserted into the guidewire lumen may be displaced to the rear side of the catheter. In this case, the stylet protrudes from a rear side guidewire port, with a rear end of the stylet coming into contact with the connector holder. Therefore, the vicinity of the rear side guidewire port may be damaged when, for example, the stylet is bent away from the outer shaft of the catheter.

Starting from a storage device as known from US 5,352,236, an object of the invention is to provide a storage device for a balloon catheter, capable of protecting the balloon catheter from damage by a rear end of a stylet protruding from a rear side guidewire port of a guidewire lumen of the balloon catheter and which prevents the guidwire lumen from collapsing along its overall length.

The above object is achieved by an arrangement of a storage device and a balloon catheter having the features of claim 1. Advantageous embodiments of such an arrangement of a storage device and a balloon catheter are subject matter of dependent claims.

### Brief Description of Drawings

The foregoing and other features, aspects and advantages of the invention will become more apparent from the following detailed description when taken in conjunction with the accompanying drawings.
Fig. 1 is a view illustrating an entire balloon catheter to be stored in a storage device for a balloon catheter according to a first embodiment.
Fig. 2 is a view illustrating an accommodating device used together with the storage device for a balloon catheter according to the first embodiment.
Fig. 3 is a view illustrating the inside of the portion A of Fig. 2.
Fig. 4 is a view illustrating the vicinity of a rear side guidewire port with a stylet inserted into the balloon catheter of Fig. 1.
Fig. 5 is a view illustrating a fixture according to the first embodiment.
Fig. 6 is a view illustrating a fixture according to a second embodiment.
Fig. 7 is a view illustrating a fixture according to a third embodiment.

### Description of Embodiments

Preferred embodiments of the present invention will be described below with reference to the accompanying drawings, in which like reference characters designate similar or identical parts throughout the several views thereof.
<1> In the storage device for a balloon catheter according to the present invention, the fixture fixes the stylet to the balloon cover by holding the stylet at a position apart from the tip portion of the balloon catheter. Consequently, the fixture can regulate the rear end position of the stylet with respect to the rear side opening of the guidewire lumen of the balloon catheter. This can prevent the balloon catheter from being pulled out of an accommodating device with the rear end of the stylet protruding from the balloon catheter.
   Therefore, it is possible to prevent, as much as possible, the rear end of the stylet protruding from the rear side opening of the guidewire lumen of the balloon catheter from bending upon contact with a connector holder of the accommodating device and, consequently, damaging the vicinity of the rear side opening of the guidewire lumen of the balloon catheter.
   In addition, a predetermined distance is maintained between the fixture and the tip portion of the balloon catheter. This can also prevent, as much as possible, the tip portion of the balloon catheter from being damaged upon contact with the fixture.
<2> In a preferred aspect of the present invention, the fixture is integrated with the balloon cover. This enables the balloon cover and the fixture to be attached to the balloon catheter at the same time, and enables the balloon cover and the fixture to be detached from the balloon catheter at the same time. Therefore, the storage device can be quickly fitted onto the balloon catheter. Furthermore, when using the balloon catheter, the storage device can be quickly detached therefrom, enabling prompt start of an operation using the balloon catheter. Also, the decreased number of parts makes the storage device easy to manage.
<3> Alternatively, the fixture may be is separable from the balloon cover. Therefore, even after the storage device is detached, the balloon cover alone can be used again. That is, the reuse of the balloon cover makes it possible to appropriately refold the dilated balloon.

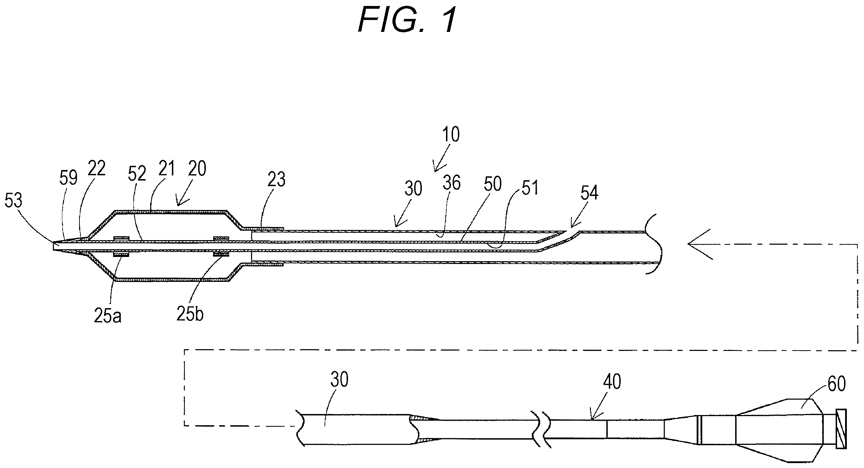

Before describing the storage device for a balloon catheter according to a present embodiment, an outline of a rapid exchange type balloon catheter to be used with the storage device for a balloon catheter according to the present embodiment will be described with reference to Fig. 1. Note that in Fig. 1, a part of the configuration is illustrated in an exaggerated manner for easy understanding of each constituent element.

In Fig. 1, the left side shown is the front side (distal side) to be inserted into a body, and the right side is the rear side (proximal side, base end side) to be operated by an operator such as a physician.

A balloon catheter 10 is used, for example, to treat an occlusion, a stenosis, or the like of a vessel in the heart. The entire length of the balloon catheter 10 is about 1500 mm.

The balloon catheter 10 mainly includes a balloon 20, a front side outer shaft 30, a rear side outer shaft 40, an inner shaft 50, and a connector 60.

The front side outer shaft 30 is a flexible tubular member formed of a resin tube.

The inner shaft 50 is coaxially arranged inside the front side outer shaft 30. The inner shaft 50 is a tubular member formed of the same resin as the resin tube constituting the front side outer shaft 30. The inner shaft 50 includes therein a guidewire lumen 51 through which a guidewire is inserted. The gap between an inner circumferential surface of the front side outer shaft 30 and an outer circumferential surface of the inner shaft 50 forms a front side dilating lumen 36, through which liquid such as a contrast agent or saline for dilating the balloon 20 is flowed.

A rear end of the inner shaft 50 is connected to a side surface of the front side outer shaft 30, thereby forming a rear side guidewire port 54 (rear side opening).

To make the balloon catheter 10 more flexible toward a front end thereof, the following has been generally practiced to, for example, change the stiffness of the balloon catheter 10. That is, a metal core shaft with a diameter reduced toward its front end is arranged in a portion ranging from a rear end of the front side outer shaft 30 to the vicinity of the rear side guidewire port 54. Alternatively, a portion of the front side outer shaft 30 on the rear side of the vicinity of the rear side guidewire port 54 is made of resin harder than the resin forming a front side portion of the front side outer shaft 30.

The inner shaft 50 has, at a front end thereof, an extension part 52 extending from a front end of the front side outer shaft 30. The extension part 52 has a tip 59 at a front end thereof.

The tip 59 is a member having a tapered outer shape, with an outer diameter gradually reduced toward a front end thereof, and is made of a flexible resin. The tip 59 is a cylindrical member constituting a front end portion of the guidewire lumen 51 and has a front side guidewire port 53 (front side opening) at a front end thereof.

The balloon 20 is a member made of resin. The balloon 20 has a dilated part 21 for dilating the balloon 20 at the center in the axial direction thereof. The balloon 20 also has a front end attachment part 22 and a rear end attachment part 23 at the front and rear sides thereof, respectively.

The front end attachment part 22 is firmly attached to a tip portion of the extension part 52 of the inner shaft 50.

The rear end attachment part 23 is firmly attached to the front end of the front side outer shaft 30. In the present embodiment, the rear end attachment part 23 is firmly attached to an outer circumferential surface of the front end of the front side outer shaft 30.

A pair of radiopaque markers 25a and 25b, spaced apart from each other by a predetermined distance, is attached to the portion of the extension part 52 of the inner shaft 50 inside the dilated part 21 of the balloon 20.

The rear side outer shaft 40 is a tubular member made of metal such as stainless steel, a so-called hypotube, having therein a rear side dilating lumen. A tip portion of the rear side outer shaft 40 is inserted into, and firmly attached to, a rear end portion of the front side outer shaft 30. As a result, the rear side dilating lumen is in communication with the front side dilating lumen 36 of the front side outer shaft 30.

The connector 60 is attached to a rear end of the rear side outer shaft 40. An indeflator (not shown) attached to the connector 60 supplies liquid for dilating the balloon 20. Consequently, the liquid flows into the balloon 20 through the rear side dilating lumen and the front side dilating lumen 36, thereby dilating the balloon 20.

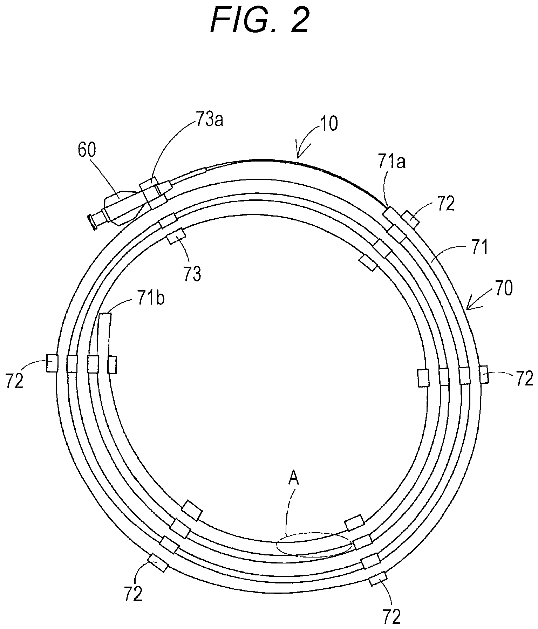

Fig. 2 is a view illustrating an accommodating device 70 in which the balloon catheter 10 described above is accommodated. The accommodating device 70 is formed of a circularly-wound tubular member 71 made of resin. In the present embodiment, the accommodating device 70 is wound three times.

In this triply-wound shape, radially adjacent portions of the tubular member 71 are connected to each other by a plurality of coupling members 72 and 73. With this configuration, the triply-wound shape is maintained. Each of the coupling members 72 and 73 has grooves each having an inner diameter substantially equal to an outer diameter of the tubular member 71. The tubular member 71 is fitted in each groove, thereby maintaining the shape of the tubular member 71.

The coupling member 73 has, at an outermost portion thereof, a connector holder 73a for holding the connector 60 of the balloon catheter 10. The connector holder 73a has a groove having an inner diameter substantially equal to an outer diameter of a cylindrical portion of the connector 60. The cylindrical portion of the connector 60 is fitted in this groove, whereby the connector 60 is held by the accommodating device 70.

The balloon catheter 10 is accommodated in the accommodating device 70 having such a configuration in such a manner that the balloon catheter 10, stored in a later-mentioned storage device 100, is inserted into the tubular member 71 from a rear end 71 a toward a front end 71b thereof. The connector 60 is then detachably held in the connector holder 73a of the coupling member 73.

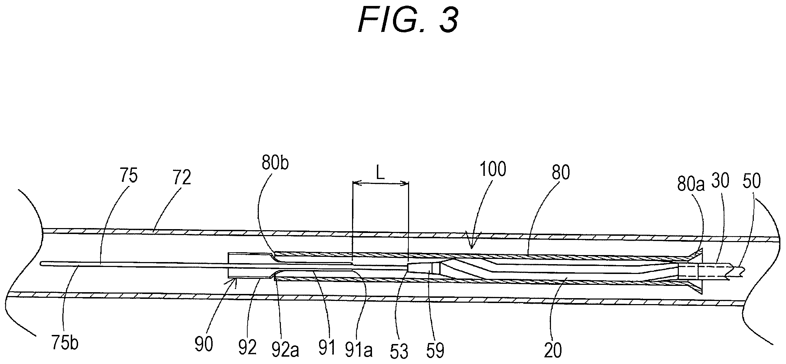

The front end of the balloon catheter 10, accommodated in the accommodating device 70 together with the storage device 100, is located at the portion A of Fig. 2. Fig. 3 illustrates the inside of the portion A.

The storage device 100 for a balloon catheter according to the present embodiment includes a stylet 75, a balloon cover 80, and a fixture 90.

As illustrated in Fig. 3, the balloon 20 of the balloon catheter 10 before use is segmented into a plurality of (three in the present embodiment) pieces and folded to be wrapped around the extension part 52 of the inner shaft 50. The folded balloon 20 is inserted into the balloon cover 80. The balloon cover 80 is a cylindrical member made of resin and having an inner diameter slightly larger than an outer diameter of the folded balloon 20. The balloon cover 80 has, at a rear end thereof, a wide opening 80a for facilitating insertion of the balloon 20.

The resin forming the balloon cover 80 is not particularly limited, but a low-friction resin is preferable in view of insertion and removal of the balloon 20. In the first embodiment, polytetrafluoroethylene is used as the resin forming the balloon cover 80.

A front end of the balloon cover 80 is provided with a distal portion 80b that contacts with the later-mentioned fixture 90.

The balloon cover 80 has not only a portion surrounding the balloon 20 of the balloon catheter 10, but also a portion further extending toward the front end thereof. That is, the balloon cover 80 has an axial length long enough to surround the entire tip portion of the balloon catheter 10 including the balloon 20 and the tip 59, to enable a holding portion 91 of the later-mentioned fixture 90 to be inserted, and to secure a length L between an end (front end) 91 a of the holding portion 91 and the front end of the tip 59.

The stylet 75 is inserted into the inner shaft 50 of the balloon catheter 10 before use. The stylet 75 prevents bending of the tip portion of the balloon catheter 10 or kinking the guidewire lumen 51 when the balloon catheter 10 is taken out from the accommodating device 70. The stylet 75 is a metal wire material having a circular cross section. In the present embodiment, the stylet 75 is made of stainless steel. The length of the stylet 75 is about 320 mm in the present embodiment, although it varies depending on the length of the inner shaft 50.

A front side of the stylet 75 extends for a predetermined distance from the front side guidewire port 53 of the balloon catheter 10. The relative positions of the stylet 75 and the balloon catheter 10 are regulated by the fixture 90. In the present embodiment, the stylet 75 is set to extend for about 70 mm from the front side guidewire port 53.

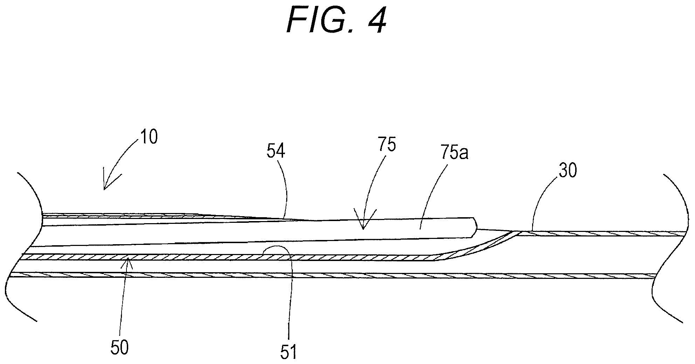

When the stylet 75 is positioned in this manner, a rear end 75a of the stylet 75 is positioned at the rear side guidewire port 54, as illustrated in Fig. 4. Note that the protruding length of the rear end 75a of the stylet 75 from the rear side guidewire port 54 is preferably 2.0 mm or less, for the purpose of minimizing damage of the rear side guidewire port 54.

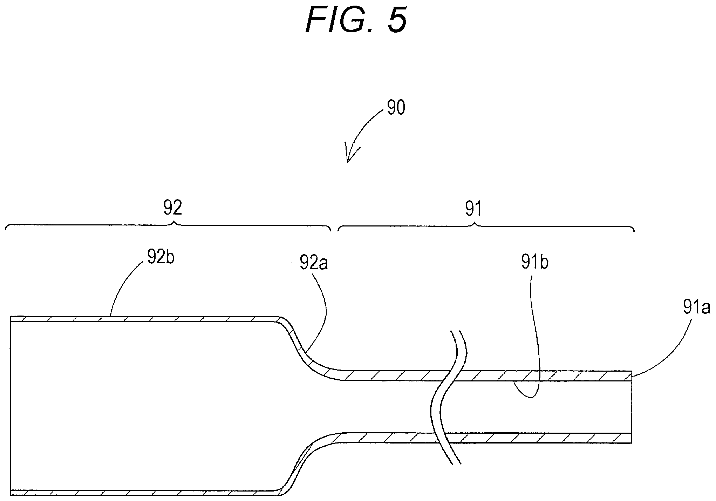

The fixture 90 is a cylindrical member made of resin. As illustrated in Fig. 5, the fixture 90 mainly includes two parts, i.e., the holding portion 91 and a regulating portion 92. The holding portion 91 is closely attached to the stylet 75. The regulating portion 92 has a large-diameter part 92b and a contact surface 92a. An outer diameter of the large-diameter part 92b is larger than that of the holding portion 91. A diameter of the contact surface 92a is reduced so as to connect the large-diameter part 92b and the holding portion 91.

The resin forming the fixture 90 is not particularly limited. In the first embodiment, polytetrafluoroethylene is used as the resin forming the fixture 90.

The fixture 90 is formed of a resin tube having the same diameter as the large-diameter part 92b of the regulating portion 92. A core rod is inserted into the resin tube and then thermally shrinking the resin tube. That is, the portion of the resin tube shrunk by heat until closely contacting with the core rod becomes the cylindrical holding portion 91 having an inner circumferential surface 91b closely contacting with an outer circumferential surface of the stylet 75. The portion not shrunk, on the other hand, becomes the large-diameter part 92b of the regulating portion 92. The contact surface 92a of the regulating portion 92 is formed between the holding portion 91 and the large-diameter part 92b.

An outer diameter of the core rod used herein is slightly smaller than that of the stylet 75. With this configuration, the fixture 90 attached onto the stylet 75 is not easily displaced on the stylet 75.

As is apparent from the above forming method, an inner diameter of the holding portion 91 is slightly smaller than the outer diameter of the stylet 75, for the purpose of close contact between the holding portion 91 and the stylet 75 used. The axial length of the holding portion 91 is not particularly limited, as long as the fixture 90 firmly fixes the stylet 75. In this respect, the predetermined distance L is preferably secured between the end 91a of the holding portion 91 and the front side guidewire port 53, which is the front end of the tip 59. This is for protecting the tip 59 from damage which would otherwise be caused upon contact between the end 91 a of the holding portion 91 and the tip 59 of the balloon catheter 10 when the fixture 90 is contacted with the balloon cover 80 as illustrated in Fig. 3.

In the present embodiment, the axial length of the holding portion 91 is about 10.0 mm, in which case the distance L is set to about 10 to 20 mm.

The outer diameter of the large-diameter part 92b of the regulating portion 92 needs to be large enough to enable the contact surface 92a of the regulating portion 92 to contact with the distal portion 80b of the balloon cover 80 when the holding portion 91 is inserted into the balloon cover 80, as illustrated in Fig. 3. In addition, the outer diameter of the large-diameter part 92b is preferably much smaller than an inner diameter of the tubular member 71, to enable the large-diameter part 92b to be accommodated in the tubular member 71 of the accommodating device 70. Therefore, the large-diameter part 92b has substantially the same outer diameter as the balloon cover 80 accommodated in the tubular member 71.

Generally, the size of the balloon cover 80 varies depending on the size of the balloon 20. The outer diameter of the large-diameter part 92b in the present embodiment is set to 1.0 mm or longer so that the fixture 90 can be used in common for the balloon cover 80 of any size.

The axial length of the regulating portion 92 is not particularly limited, but is preferably about 5.0 mm or longer for easy insertion and removal of the fixture 90. On the other hand, the axial length of the regulating portion 92 is preferably not too long because too long a length thereof increases the area of the regulating portion 92 coming into contact with an inner wall of the tubular member 71 of the accommodating device 70 when the balloon catheter 10 is pulled out of the accommodating device 70.

Based on the above configuration, a method of using the storage device 100 for a balloon catheter according to the first embodiment will be described.

Before the balloon catheter 10 is manufactured and put in a sterilized bag, the balloon 20 is segmented into a plurality of pieces, folded to be wound around the extension part 52 of the inner shaft 50, and inserted into the balloon cover 80 as illustrated in Fig. 3. The stylet 75 is inserted into the guidewire lumen 51 of the inner shaft 50. As described above, the stylet 75 extends for a predetermined length from the front side guidewire port 53. From this state, a front end 75b of the stylet 75 is inserted into the holding portion 91 of the fixture 90 with the end 91 a of the holding portion 91 facing the tip 59 of the balloon catheter 10.

The fixture 90 is inserted into the balloon cover 80 until the contact surface 92a of the regulating portion 92 contacts with the distal portion 80b of the balloon cover 80. At this time, the fixture 90 is positioned such that the end 91 a of the holding portion 91 is apart from the front side guidewire port 53, which is the end of the tip 59, by the predetermined distance L.

In this case, the stylet 75 is positioned such that the rear end 75a thereof is located at the rear side guidewire port 54, as illustrated in Fig. 4.

Note that the folded balloon 20 is inserted into the balloon cover 80 so as to closely contact therewith. An outer surface of the balloon 20 comes into even closer contact with an inner surface of the balloon cover 80 due to, for example, depressurization in the final sterilization. Therefore, the balloon cover 80 is hardly displaced with respect to the balloon 20.

Under the above conditions, the balloon catheter 10 is inserted into the accommodating device 70 from the rear end 71 a of the tubular member 71. The connector 60 is then fixed in the connector holder 73 a of the coupling member 73. In this state, the balloon catheter 10 is put in the sterilized bag.

The balloon catheter 10 thus manufactured is accommodated in the accommodating device 70, with the fixture 90 in contact with the balloon cover 80. Therefore, it is possible to prevent the stylet 75 from being displaced toward the rear end of the balloon catheter 10. In this state, the distance L is maintained between the end 91 a of the holding portion 91 of the fixture 90 and the front side guidewire port 53, which is the end of the tip 59. Consequently, the front end of the fixture 90 does not come into contact with the tip 59 of the balloon catheter 10. Therefore, despite the fixture 90, the tip 59 of the balloon catheter 10 is not damaged thereby.

When taking out the balloon catheter 10 from the sterilized bag to use it, an operator such as a physician detaches the connector 60 from the connector holder 73a of the accommodating device 70. The operator then pulls the balloon catheter 10 out of the tubular member 71. At this time, the balloon catheter 10 is pulled out while contacting with the inner wall surface of the tubular member 71. However, since the distal portion 80b of the balloon cover 80 contacts with the contact surface 92a of the fixture 90, it is possible to prevent the stylet 75 from being displaced toward the rear end of the balloon catheter 10. It is thus possible to prevent the balloon catheter 10 from being pulled out of the tubular member 71 of the accommodating device 70 with the rear end 75a of the stylet 75 protruding from the balloon catheter 10. Therefore, it is possible to prevent, as much as possible, the stylet 75 protruding from the rear side guidewire port 54 from bending upon contact with the connector holder 73a of the accommodating device 70 and, consequently, damaging the vicinity of the rear side guidewire port 54.

The balloon catheter 10 thus pulled out is used by the operator with both of the stylet 75 and the fixture 90 removed. The fixture 90 has the large-diameter part 92b, which enables the operator to easily remove the fixture 90 and the stylet 75 at the same time by pinching the large-diameter part 92b and pulling them from the balloon catheter 10.

In this manner, only the fixture 90 and the stylet 75 can be removed, with the balloon cover 80 left on the balloon 20.

Note that all the members of the storage device 100 can be removed at the same time by sliding the balloon cover 80 forward. More specifically, since the distal portion 80b of the balloon cover 80 is in contact with the contact surface 92a of the fixture 90, sliding the balloon cover 80 as described above makes the balloon cover 80, the fixture 90, and the stylet 75 move together while remaining integrated with one another. As a result, the balloon cover 80, the fixture 90, and the stylet 75 can be removed at the same time.

The balloon catheter 10 with the balloon cover 80, the fixture 90, and the stylet 75 thus removed is inserted into a tubular cavity inside the body, for the treatment in which a stenosis inside a vessel is dilated with the balloon 20, for example.

After the treatment using the balloon catheter 10 is finished, the balloon catheter 10, taken out of the body with the balloon 20 deflated, may be inserted into the body and used again when, for example, the stenosis is not sufficiently dilated. In this case, the operator needs to appropriately fold the balloon 20 again, which may require reuse of the balloon cover 80. The fixture 90 in the present embodiment is separate from the balloon cover 80. Furthermore, no additional special member is attached to the balloon cover 80. Therefore, the balloon 20 can be appropriately folded again using only the balloon cover 80 according to the normal procedure.

As described above, the storage device 100 for a balloon catheter according to the present embodiment prevents the stylet 75 from being displaced toward the rear side guidewire port 54 of the balloon catheter 10. It is thus possible to prevent the balloon catheter 10 from being pulled out of the tubular member 71 of the accommodating device 70 with the rear end 75a of the stylet 75 protruding from the balloon catheter 10. Therefore, it is possible to prevent, as much as possible, the stylet 75 protruding from the rear side guidewire port 54 from bending upon contact with the connector holder 73a of the accommodating device 70 and, consequently, damaging the vicinity of the rear side guidewire port 54.

The regulating portion 92 maintains the predetermined distance between the end 91 a of the holding portion 91 and the tip 59 of the balloon catheter 10. This can prevent, as much as possible, the tip 59 from being damaged upon contact with the end 91 a of the holding portion 91.

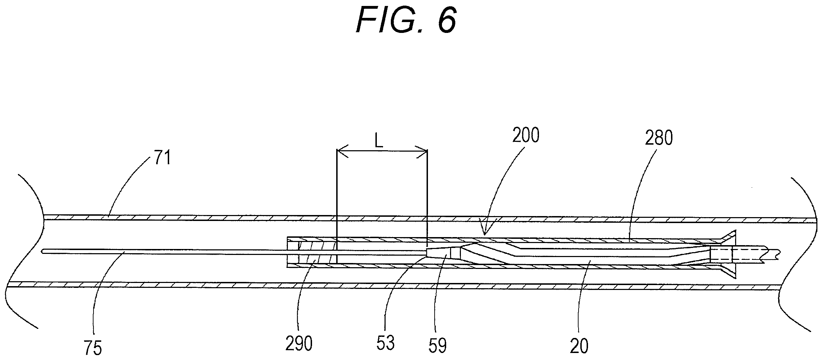

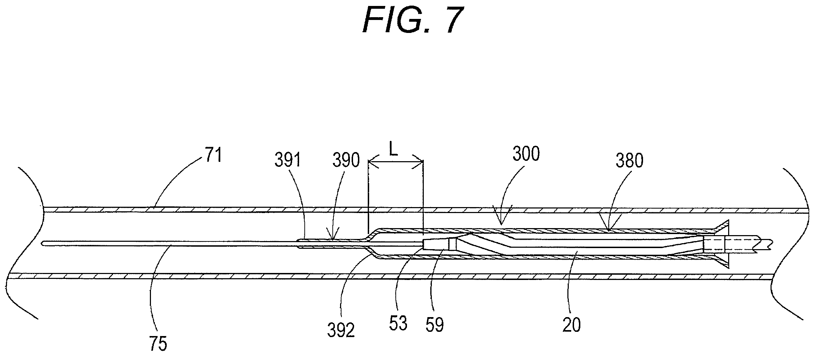

In the storage device 100 for a balloon catheter according to the embodiment described above, the fixture 90 is separate from the balloon cover 80. Alternatively, however, the fixture may be integrated with the balloon cover in the storage device, as in second and third embodiments illustrated in Figs. 6 and 7, respectively.

In a storage device 200 for a balloon catheter according to the second embodiment illustrated in Fig. 6, a fixture 290 (as well as a stylet 75) and a balloon cover 280 are integrated in such a manner that an adhesive is filled in between the stylet 75 and the balloon cover 280 after the balloon cover 280 is fitted onto a balloon 20. The fixture 290 in this embodiment is formed of the adhesive, which facilitates the formation thereof. Furthermore, the stylet 75 can be firmly bonded to the fixture 290.

In a storage device 300 for a balloon catheter according to the third embodiment illustrated in Fig. 7, a fixture 390 (as well as a stylet 75) and a balloon cover 380 are integrated by thermally shrinking a distal portion of the balloon cover 380 after the balloon cover 380 is fitted onto a balloon 20. The storage device 300 has the fixture 390 and the balloon cover 380 at the front and rear sides thereof, respectively. The fixture 390 has a holding portion 391 at a front end thereof, and a transitional part between the holding portion 391 and the balloon cover 380 at the rear side of the fixture 390 serves as a regulating portion 392.

In this configuration, the fixture 390 is firmly attached to the stylet 75 by being thermally shrunk. Therefore, the fixture 390 and the stylet 75 can be firmly bonded to each other. Furthermore, the storage device 300 has a small number of parts, which makes the device inexpensive and easy to manage.

In the embodiments described above, the storage device for a balloon catheter is applied to the balloon catheter 10 used for the treatment of a vessel in the heart. Alternatively, however, the storage devices for a balloon catheter according to the first to third embodiments can be applied to a balloon catheter used for various other operations.

## Claims

1. An arrangement of a storage device (100, 200, 300) and a rapid exchange type balloon catheter (10) having a folded balloon (20),
wherein the balloon catheter (10) further includes an inner shaft (50) which defines a guidewire lumen (51) and forms at its rear end a rear side guidewire port (54) and at its front end a front side guidewire port (53), and
wherein the storage device (100, 200, 300) comprises:
a stylet (75) inserted into the guidewire lumen (51) of the balloon catheter (10);
a balloon cover (80, 280, 380) surrounding the folded balloon (20) of the balloon catheter (10); and
a fixture (90, 290, 390) attached to the stylet (75) and cooperating with a front end of the balloon cover (80, 280, 380) so as to prevent the stylet (75) from being displaced toward a rear end of the balloon cover (80, 280, 380), **characterized in that**
the stylet (75) is inserted into the inner shaft (50) of the balloon catheter (10) so that a front side of the stylet (75) extends for a predetermined distance from the front side guidewire port (53) of the balloon catheter (10) and a rear end (75a) of the stylet (75) is positioned at the rear side guidewire port (54), and
the fixture (90, 290, 390) attached to the stylet (75) is spaced by a predetermined distance (L) from the front side guidewire port (53) of the balloon catheter (10).

2. The arrangement according to claim 1, wherein
the inner shaft (50) is coaxially arranged inside a front side outer shaft (30) of the balloon catheter (10), and
the rear end of the inner shaft (50) is connected to a side surface of the front side outer shaft (30), thereby forming the rear side guidewire port (54).

3. The arrangement according to claim 1 or 2, wherein the fixture (290, 390) is integrated with the balloon cover (280, 380).

4. The arrangement according to claim 1 or 2, wherein the fixture (90) is separable from the balloon cover (80).

5. The arrangement according to one of claims 1 to 4, wherein the fixture (90) includes a holding portion (91) closely attaching to the stylet (75) and a regulating portion (92).

6. The arrangement according to claim 5, wherein
the regulating portion (92) includes a large-diameter part (92b) and a contact surface (92a),
the large-diameter part (92b) has a larger diameter than the holding portion (91), and
the contact surface (92a) has a reduced diameter so as to connect the large-diameter part (92b) and the holding portion (91).

## Patentansprüche

1. Anordnung aus einer Lagervorrichtung (100, 200, 300) und einem Schnellwechsel-Ballonkatheter (10) mit einem zusammengefalteten Ballon (20),
wobei der Ballonkatheter (10) weiter einen Innenschaft (50) aufweist, der ein Führungsdraht-Lumen (51) definiert und an seinem hinteren Ende einen hinteren Führungsdraht-Port (54) und an seinem vorderen Ende einen vorderen Führungsdraht-Port (53) bildet, und
wobei die Lagervorrichtung (100, 200, 300) aufweist:
ein in das Führungsdraht-Lumen (51) des Ballonkatheters (10) eingeführtes Stilett (75);
eine den zusammengefalteten Ballon (20) des Ballonkatheters (10) umschließende Ballonhülse (80, 280, 380); und
eine an dem Stilett (75) angebrachte Vorrichtung (90, 290, 390), die mit dem vorderen Ende der Ballonhülse (80, 280, 380) zusammenwirkt, um eine Verschiebung des Stilett (75) in Richtung des hinteren Endes der Ballonhülse (80, 280, 380) zu verhindern, **dadurch gekennzeichnet, dass**
das Stilett (75) in den Innenschaft (50) des Ballonkatheters (10) so eingeführt ist, dass seine vordere Seite um eine vorgegebene Länge aus dem vorderen Führungsdraht-Port (53) des Ballonkatheters (10) hervorragt und sein hinteres Ende (75a) in dem hinteren Führungsdraht-Port (54) angeordnet ist, und
die an dem Stilett (75) angebrachte Vorrichtung (90, 290, 390) in einem vorgegebenen Abstand (L) von dem vorderen Führungsdraht-Port (53) des Ballonkatheters (10) angeordnet ist.

2. Anordnung nach Anspruch 1, wobei
der Innenschaft (50) in einem vorderen Außenschaft (30) des Ballonkatheters (10) koaxial angeordnet ist, und
das hintere Ende des Innenschafts (50) mit einer Seitenfläche des vorderen Außenschafts (30) verbunden ist und dadurch den hinteren Führungsdraht-Port (54) bildet.

3. Anordnung nach Anspruch 1 oder 2, wobei die Vorrichtung (290, 390) in die Ballonhülse (280, 380) integriert ist.

4. Anordnung nach Anspruch 1 oder 2, wobei die Vorrichtung (90) von der Ballonhülse (80) trennbar ist.

5. Anordnung nach einem der Ansprüche 1 bis 4, wobei die Vorrichtung (90) einen mit dem Stilett (75) eng verbundenen Halteabschnitt (91) und einen Positionierabschnitt (92) aufweist.

6. Anordnung nach Anspruch 5, wobei
der Positionierabschnitt (92) einen im Durchmesser großen Teil (92b) und eine Kontaktfläche (92a) aufweist,
der im Durchmesser große Teil (92b) einen größeren Durchmesser hat als der Halteabschnitt (91), und
die Kontaktfläche (92a) einen reduzierten Durchmesser hat, um den im Durchmesser großen Teil (92b) mit dem Halteabschnitt (91) zu verbinden.

## Revendications

1. Agencement d'un dispositif de stockage (100, 200, 300) et d'un cathéter à ballonnet de type à échange rapide (10) ayant un ballonnet plié (20),
dans lequel le cathéter à ballonnet (10) comporte en outre un arbre interne (50) qui définit une lumière de fil-guide (51) et forme au niveau de son extrémité arrière un orifice de fil-guide côté arrière (54) et au niveau de son extrémité avant un orifice de fil-guide côté avant (53), et
dans lequel le dispositif de stockage (100, 200, 300) comprend :
un stylet (75) inséré dans la lumière de fil-guide (51) du cathéter à ballonnet (10) ;
une couverture de ballonnet (80, 280, 380) entourant le ballonnet plié (20) du cathéter à ballonnet (10) ; et
un dispositif de fixation (90, 290, 390) attaché au stylet (75) et coopérant avec une extrémité avant de la couverture de ballonnet (80, 280, 380) de façon à empêcher le déplacement du stylet (75) vers une extrémité arrière de la couverture de ballonnet (80, 280, 380), **caractérisé en ce que**
le stylet (75) est inséré dans l'arbre interne (50) du cathéter à ballonnet (10), de sorte qu'un côté avant du stylet (75) s'étende d'une distance prédéterminée à partir de l'orifice de fil-guide côté avant (53) du cathéter à ballonnet (10) et qu'une extrémité arrière (75a) du stylet (75) soit positionnée au niveau de l'orifice de fil-guide côté arrière (54), et
le dispositif de fixation (90, 290, 390) attaché au stylet (75) est espacé d'une distance prédéterminée (L) de l'orifice de fil-guide côté avant (53) du cathéter à ballonnet (10).

2. Agencement selon la revendication 1, dans lequel
l'arbre interne (50) est agencé coaxialement à l'intérieur d'un arbre externe côté avant (30) du cathéter à ballonnet (10), et
l'extrémité arrière de l'arbre interne (50) est raccordée à une surface de côté de l'arbre externe côté avant (30), formant ainsi l'orifice de fil-guide côté arrière (54).

3. Agencement selon la revendication 1 ou 2, dans lequel le dispositif de fixation (290, 390) est intégré à la couverture de ballonnet (280, 380).

4. Agencement selon la revendication 1 ou 2, dans lequel le dispositif de fixation (90) peut être séparé de la couverture de ballonnet (80).

5. Agencement selon l'une des revendications 1 à 4, dans lequel le dispositif de fixation (90) comporte une portion de maintien (91) s'attachant étroitement au stylet (75) et une portion de régulation (92).

6. Agencement selon la revendication 5, dans lequel
la portion de régulation (92) comporte une partie de grand diamètre (92b) et une surface de contact (92a),
la partie de grand diamètre (92b) a un diamètre plus grand que la portion de maintien (91), et
la surface de contact (92a) a un diamètre réduit de façon à raccorder la partie de grand diamètre (92b) et la portion de maintien (91).
